# EUROPEAN PATENT SPECIFICATION

(11) **EP 4 260 793 B1**
(45) Date of publication and mention of the grant of the patent: **22.04.2026**
(21) Application number: 20851281.4
(22) Date of filing: 07.12.2020
(51) Int. Cl.: A61B 3/10, A61F 2/14, A61F 2/16

(54) **COMPUTER-IMPLEMENTED METHOD FOR CALCULATING SURGICALLY INDUCED ASTIGMATISM**
COMPUTERIMPLEMENTIERTES VERFAHREN ZUR BERECHNUNG VON CHIRURGISCH INDUZIERTEM ASTIGMATISMUS
PROCÉDÉ MIS EN OEUVRE PAR ORDINATEUR

(43) Date of publication of application: 18.10.2023
(73) Proprietor: Centro de Oftalmologia Barraquer, S.A., 08021 Barcelona (ES); Pesic, Milan, 08021 Barcelona (ES); Salvador Gómez, Sergio, 08027 Barcelona (ES)
(72) Inventor: PESIC, Milan, 08021 BARCELONA (ES); SALVADOR GÓMEZ, Sergio, 08027 BARCELONA (ES); BARRAQUER COMPTE, Rafael, 08021 BARCELONA (ES); FERNÁNDEZ ROSES, Joaquim, 08191 BARCELONA (ES)
(74) Representative: Isern Patentes y Marcas S.L.
(86) International application number: PCT/ES2020/070771
(87) International publication number: WO 2022/123091

(56) References cited:
- RU-C1- 2 165 748
- US-A1- 2014 211 151
- US-B2- 7 476 248

## Description

### TECHNICAL FIELD OF THE INVENTION

The present invention is related to calculating the surgically induced astigmatism when an incision is made in the corneal portion, the sclera or corneal limbus of the eye during a surgery, being particularly related to calculating the astigmatism surgically induced by an incision made in the cornea for the implantation of an intraocular lens as a replacement for the crystalline lens in a surgical intervention for correcting cataracts, as well as to calculating the toricity of said intraocular lens.

### STATE OF THE ART

In the vast majority of eye surgeries wherein a corneal incision is made, such an incision leads to an astigmatism induced by the surgery itself, which is called a Surgically Induced Astigmatism (hereinafter, throughout this document to refer to such astigmatism, SIA will be used). This means that the patient, after the surgery, may exhibit postoperative astigmatism resulting from the sum of a prior or preoperative astigmatism, which they had before the surgery if this refractive error had been present, and the SIA corresponding to surgery.

Thus, for example, in a surgical method for correcting cataracts, a cataract being an eye defect wherein the natural lens of the eye degenerates, blurring vision, the surgeon makes an incision in the cornea in order to remove the crystalline lens, replacing it with a suitable intraocular lens (IOL). Consequently, such an intervention generates an SIA relating to the incision.

It should be noted that for these types of cataract correction methods, since the surgeon must implant an IOL in order to replace the crystalline lens, it is intended that this IOL corrects all types of refractive errors that the patient may have previously (myopia, hyperopia and/or astigmatism).

Since, as already mentioned, the surgical method for correcting cataracts involves the generation of an SIA on the patient, this means that the IOL that should be used in most cases is of the toric type so that, in addition to correcting refractive errors such as myopia or hyperopia existing before the operation in the patient, the preoperative astigmatism and the SIA can be corrected.

Thus, it is common for the surgeon to implant a toric monofocal, toric bifocal, toric multifocal, EDOF (Extended Depth of Focus) IOL and any other type of IOL that may appear on the market that is associated with correcting astigmatism in order to try to correct after the cataract surgery all the refractive errors that the patient had before the operation and the corresponding SIA generated by the cataract surgery method.

Currently there are a multitude of calculators developed by the IOL manufacturers themselves that, by entering certain parameters, are able to provide the surgeon responsible for the cataract surgery with the most suitable IOL, within which they manufacture together with the corresponding toricity, in order to correct both the SIA and the other refractive errors of the patient before the surgery.

However, it has been found that the IOLs provided by these calculators have a common problem consisting in that they do not adequately correct the refractive error of the astigmatism. Specifically, if the patient did not have astigmatism before surgery, they will have a certain level of astigmatism after the operation, induced by the incision, which prevents them from seeing reality correctly without using suitable lenses for the correction thereof. Likewise, if the patient had astigmatism before surgery, they continue to have astigmatism after the operation, maybe with a lower level than what they had before the operation, but preventing them from seeing reality correctly without needing suitable lenses.

Therefore, none of these calculators are capable of predicting the postoperative SIA and, therefore, are not able to determine the suitable toricity of the IOL to be applied in order to correct it, since in reality they leave such a parameter to the discretion of the surgeon. In other words, all these calculators have an input variable consisting of the SIA that the surgeon introduces into the calculator, for which reason they are not actually capable of calculating it and hence why they are not reliable in relation to this parameter.

Accordingly, it becomes necessary in any method for correcting visual defects that involves making an incision in the cornea, and very especially in the surgical method for correcting cataracts, to apply a computer-implemented method which enables the SIA associated with the incision caused by the surgery to be determined with precision.

US7476248, RU2165748 and US2014/211151 disclose example of prior art calculations of surgically induced astigmatism.

### DESCRIPTION

In order to overcome the drawbacks found, the present invention provides a computer-implemented method adapted to calculate the astigmatism which is induced in a patient when an incision is made in the cornea, as defined in independent claim 1. Such astigmatism, called surgically induced astigmatism or SIA, in order to implant an intraocular lens with a certain size, in order to correct some visual defects such as myopia, hyperopia and/or a basic astigmatism.

Such visual defects are called preoperative visual defects since they already exist before the intervention and can be diagnosed prior to it.

The first step of the computer-implemented method consists of establishing the orientation of the steepest axis and the orientation of the flattest axis of the cornea starting from a measurement of the curvature of said cornea by means of an ophthalmic diagnostic device.

The measurement of the corneal curvature is usually carried out by projecting light onto it from an ophthalmic diagnostic device which, in practice can be a keratometer, a corneal topographer, a high-resolution scanner or similar. Regardless of the ophthalmic diagnostic device used, the required results are, at least, those corresponding to the measurement of the radius of curvature of the central portion of the cornea, that which is involved in vision, and the meridians with more and less ocular power which, as is known, are usually perpendicular to each other, in the case of dealing with virgin or healthy corneas, in other words, those corneas that have not undergone any previous surgery or pathology. Through these measurements it is possible to determine the orientation of the steepest axis and the flattest axis of the cornea, which is necessary in order to carry out subsequent steps of the method of the invention. Likewise, it should be noted that said ophthalmic diagnostic devices also make it possible to obtain corneal asphericity (Q), the value of which may be relevant in order to calculate the SIA since such corneal asphericity can produce a change in the SIA.

Next, within the computer-implemented method, it is necessary to obtain an incision position, which must be between at least the orientation defined for the steepest axis or the orientation defined for the flattest axis. In other words, the incision to be made in the cornea will be located in at least one of said flattest axis or steepest axis. The method guarantees a certain freedom to establish the axis on which to cut, but at the same time it eliminates arbitrary incision positions that would lead to unsatisfactory and/or unpredictable results of the surgically induced astigmatism.

In other preferred embodiments, the incision position obtained may also be in an axis which is located at 45° from the steepest axis or from the flattest axis, as well as a temporal axis which coincides with the temporal bone of the patient.

It should be noted that, in the case of the healthy corneas, as mentioned earlier, the steepest axis and the flattest axis are perpendicular to each other, such that the axis which is located at 45° from the steepest axis coincides with the axis that is 45° from the flattest axis, said axis at 45° being a bisector axis between the steepest axis and the flattest axis.

Likewise, it is necessary to obtain an incision size to be made in the cornea for the implantation of the intraocular lens. The size of the incision, in a simplified manner, could be defined by the width and depth thereof. The incision width is determined by the surgical instruments arranged with the means for making such incision, for example, a sharp tip made of prismatic diamond or a needle, as well as the power and/or the design of the intraocular lens. The incision depth refers to the amount that the instrument penetrates into the cornea. According to different studies, the most stable incision size setting (width vs depth) is the square-shaped one, wherein the incision depth equals the incision width.

Preferably, the size of the incision is between 1.8 and 3 mm and, more preferably, working with three incision sizes: 2 mm, 2.2 mm and 2.8 mm.

Next, an incision architecture is obtained which is defined as the number of planes through which the incision can be extended.

In this sense, the obtained incision architecture can be carried out in at least one plane, preferably being horizontal or slightly oblique, although it can be carried out in three planes as well: perpendicular to the cornea, horizontal and inclined entering the anterior chamber of the eye. The three-plane incision architecture is made so that the incision functions like a one-way valve, producing an incision that is waterproof and self-healing.

Once the incision position, incision size and incision architecture have been obtained, the computer-implemented method of the invention proceeds to calculate the astigmatism that will be surgically induced when making the incision in the cornea under such position conditions, size and incision architecture.

According to the above and, as a remarkable advantage, the implemented method enables the user, for example, the surgeon, to be able to choose between at least two incision positions: in the steepest axis of the cornea, in the flattest axis of the cornea, even in other embodiments, being able to choose incision positions in the axis at 45° from the steepest axis or the flattest axis or in the temporal axis, in order to better adapt to the physiognomy of the patient and/or the factors thereof and, even then, obtain calculated values of the surgically induced astigmatism which are within predictable intervals.

This fact facilitates the comfort of the surgeon, which is of great importance in order for the incision in the cornea to be carried out in the best possible manner, and which enables the skills of the surgeon to be deployed, which are very necessary, once the incision has been made on the cornea, in order to implant the intraocular lens and align it with respect to the steepest axis.

For this very reason, the fact that the user can select different incision positions enables them to have a certain independence when operating and finding the position that may be most comfortable for them, either because they have greater ability in one position with respect to another, due to the arrangement of the operating room that almost forces them to operate in a certain position, or due to the very physiognomy of the patient (in certain patients with prominent nasal septums, prominent cheekbones, sunken eyes, prominent orbital rim, etc., there are certain incision positions which facilitate the surgical intervention, including the subsequent implantation of the intraocular lens).

It should be noted that in a preferred embodiment of the invention wherein the size of the incision is between 1.8 and 3 mm, for a specific incision position and a specific type of incision architecture, the surgically induced astigmatism calculated for the smallest incision size (1.8 mm) and for the largest incision size (3 mm) differ by a maximum of 0.7 D.

If the incision sizes are 2 mm, 2.2 mm and 2.8 mm, for a specific incision position and a specific type of incision architecture, the surgically induced astigmatism calculated for the incision size of 2 mm (with that specific incision position and this specific type of incision architecture) and the surgically induced astigmatism calculated for the incision size of 2.8 mm (with that same incision position and that same type of incision architecture) differ by a maximum of 0.5 D.

In another preferred embodiment of the invention, if the incision position obtained corresponds to the orientation of the steepest axis and the incision architecture obtained affects more than one plane, the surgically induced astigmatism calculated is negative, with a maximum absolute value of 0.75 D.

Likewise, in the computer-implemented method, if the incision position which is obtained corresponds to the orientation of the flattest axis and the incision architecture affects more than one plane, then the surgically induced astigmatism calculated is positive, with a maximum value of 0.55D.

Not claimed is a computer-implemented method for calculating the toricity of an intraocular lens foreseen to be implanted in a surgical method for correcting vision defects.

The first step of this method consists of obtaining a preoperative astigmatism value, which can be obtained through a diagnostic examination of possible visual defects of the patient, within which, in addition to the astigmatism, myopia or hyperopia can also be detected, and wherein at least one ophthalmic diagnostic device is involved in the diagnostic.

The next step consists of obtaining at least one intraocular lens power which can be determined at least partially by means of the ophthalmic diagnostic device once the visual diagnostic examination of the patient has been performed. Preferably, said ophthalmic diagnostic device is a biometer which, in conjunction with a specific software, enables the power of the lens to be obtained. Optionally, together with the size of the lens, the shape or design of the intraocular lens can also be determined.

Taking the above into account, the surgically induced astigmatism is then calculated according to the computer-implemented method for calculating the surgically induced astigmatism, as explained in previous paragraphs, which is produced when an incision is made in the cornea in order to introduce the intraocular lens obtained.

Having obtained the preoperative astigmatism and the surgically induced astigmatism, a suitable toricity of the intraocular lens (such intraocular lens can be from any manufacturer) is then calculated in order to correct the astigmatism resulting from the sum of the preoperative and surgically induced astigmatisms.

The main advantage of this method is that it can be guaranteed that the residual or post-operative refractive astigmatism of the patient will be practically zero, given that the toricity of the calculated intraocular lens takes into account the preoperative astigmatism and the surgically induced astigmatism. Therefore, after the surgical intervention, the patient can see without needing lenses in most cases.

The disclosed methods are preferably oriented for surgical interventions for correcting cataracts which, as is widely known, require the replacement of the crystalline lens by an intraocular lens which enables the visual capacity of the patient to be recovered.

### DETAILED DESCRIPTION OF AN EXEMPLARY EMBODIMENT

In the following detailed description, numerous specific details are set forth in the form of examples to provide a thorough understanding of the relevant teachings. However, it will be apparent to those skilled in the art that the present teachings can be put into practice without such details.

According to a preferred embodiment, is disclosed a computer-implemented method for calculating surgically induced astigmatism when an incision is made in the cornea in order to introduce an intraocular lens with a certain power and design.

In the first step of this method, an orientation of the main corneal axes must be established which, in general, refers to an orientation of the flattest axis and an orientation of the flattest axis of the cornea starting from a measurement of the curvature of said cornea by means of an ophthalmic diagnostic device.

In order to obtain the orientation of the steepest and flattest axes, a diagnostic of the cornea is performed by means of an ophthalmic diagnostic device, such as a keratometer, a corneal topographer, a high-resolution scanner, among others, wherein at least the curvature of the steepest and flattest corneal meridians must be obtained.

It is important to note that the cornea is an aspherical hemisphere with the steepest and flattest meridians thereof and accordingly comprises, as said, corneal axes, wherein the main corneal axes are called: the steepest axis, which is located in the steepest meridian, and, at 90 degrees, a flattest axis, which is located in the flattest meridian, wherein said main corneal axes intersect in the centre of the cornea. Since the cornea is a toroid, there are other corneal axes, different from the main ones, which pass through the intersection of the main corneal axes, for example, a corneal axis at 45 degrees from the steepest or flattest axis.

The next step of the method comprises obtaining an incision position between at least the orientation of the steepest axis or the orientation of the flattest axis.

In this preferred embodiment, the incision position obtained for the method, which mainly comes from the decision of where the surgeon prefers to cut, can be in the orientation of the steepest axis or in the orientation of the flattest axis.

In the case of the steepest axis or the flattest axis coinciding with the temporal axis, this particular embodiment would also enable the temporal axis as the incision position.

In another preferred embodiment, the incision position can also be in an axis at 45° from the steepest axis or from the flattest axis which, in the case of healthy corneas, will coincide.

The fact that the incision position is located in the flattest axis or the steepest axis or in the axis at 45° from any of the previous axes enables the surgically induced astigmatism that will be generated by the incision to be limited. It is well known that the degree of astigmatism that is induced by the surgery is directly related to the incision position, for which reason establishing that said position can be found in any of the steepest or flattest axes or in the axis at 45° from any of them will prevent the SIA from being outside of expected values.

Specifically, it should be noted that, of such incision positions, the ones that offer the best results are the ones obtained in the steepest axis and the flattest axis.

Now, the corneal meridians can be divided into angular portions of 30 degrees, such that, for a with-the-rule astigmatism, wherein the steepest axis is 90° or vertical, parallel to the sagittal plane, the steepest meridian of the cornea is located between 60 to 90 degrees or between 90 to 120 degrees.

For an against-the-rule astigmatism, wherein the steepest axis is the 180-degree or horizontal axis, the steepest corneal meridian will be located between 0 degrees to 30 degrees or between 150 degrees to 180 degrees.

In the case of an oblique astigmatism, wherein the steepest axis is not at 90 degrees or at 180 degrees, the steepest corneal meridian will be located between 30 degrees to 60 degrees or between 120 degrees to 150 degrees.

In this manner, preferably, the orientation of the steepest axis or the flattest axis can be obtained within the portions of [0, 30), [30, 60), [60, 120], (120, 150], (150, 180], in order to attend to the possible positions wherein the steepest axis or the flattest axis can be located.

The next step of the method consists of obtaining an incision size depending on the power of the intraocular lens and/or the size of a surgical instrument configured to carry out said incision. The type or design of intraocular lens can also be taken into account when obtaining the incision size.

As a result of the diagnostic, the ophthalmic diagnostic device suggests the use of an intraocular lens with a power and, preferably, also a design such that detected preoperative visual defects are corrected. At this power and possible design of the intraocular lens, the incision size must be adjusted, such that it enables said lens to be implanted and placed in position. The size of the incision may also be conditioned by the surgical instruments available, since incisions that are smaller than the cutting tip of the instrument cannot be made. In this embodiment of the invention, the size of the incision is between 1.8 mm to 3.0 mm, particularly, the size of the incision can be chosen between 2.0 mm, 2.2 mm and 2.8 mm.

In the next step, the method establishes obtaining an incision architecture, defined as the number of planes through which the incision in the cornea can extend. In the preferred embodiment, the incision architecture is carried out in more than one plane, which means that the surgical instruments change the orientation thereof at least one time once they have penetrated the cornea.

Next, within the method, the surgically induced astigmatism is calculated depending on the incision position obtained, the incision size obtained and the incision architecture obtained.

In the exemplary embodiment, wherein the incision position obtained is in the steepest axis or the flattest axis and the orientation of such axes will be located in the corneal meridional interval [0 to 30), [30, 60), [60, 120], (120, 150], (150, 180], the incision size can be 2.0 mm, 2.2 mm and 2.8 mm, and an incision architecture which affects more than one plane, calculations are performed obtaining specific values for the SIA which can then be used to calculate the toricity of an intraocular lens, or recalculate the toricity of a previously determined intraocular lens.

Therefore, when the incision position obtained corresponds to the most steepest axis and said steepest axis is located within the angular sector of [0, 30) degrees or (150, 180] degrees, the SIA is 0.0 D for an incision size of 2.0 mm and 2.2 mm, and -0.15 D for a lens incision size of 2.8 mm.

In the case of the orientation of the steepest axis being within the angular sector of [30, 60) degrees or (120, 150] degrees, the SIA calculated is 0.00 D for an incision size of 2.0 mm, -0.20 D for an incision size of 2.2 mm, and -0.40 D for an incision size of 2.8 mm.

Now, when the orientation of the steepest axis is located within the angular sector of [60, 120] degrees, the SIA calculated is -0.25 D for an incision size of 2.0 mm, -0.50 D for an incision size of 2.2 mm, and -0.75 D for an incision size of 2.8 mm.

Moreover, if the incision position obtained corresponds to the steepest axis and said steepest axis is located within the angular sector of [0, 30) degrees or (150, 180] degrees, the SIA calculated is 0.0 D for an incision size of 2.0 mm, 2.2 mm and 2.8 mm.

In the case of the orientation of the flattest axis being within the angular sector of [30, 60) degrees or (120, 150] degrees, the SIA calculated is 0.00 D for an incision size of 2.0 mm, 0.15 D for an incision size of 2.2 mm, and 0.30 D for an incision size of 2.8 mm.

Now, if the orientation of the flattest axis is located within the angular sector of [60, 120] degrees, the SIA calculated is 0.20 D for an incision size of 2.0 mm, 0.35 D for an incision size of 2.2 mm, and 0.55 D for an incision size of 2.8 mm.

Moreover, if in the incision architecture obtained it affects one plane, the astigmatism calculated has an absolute maximum value of 0.2 D.

Likewise, it should be noted that, regardless of the incision position obtained, when the corresponding intraocular lens is implanted by the surgeon, said lens should be oriented with respect to the steepest axis. In other words, the intraocular lens comprises markings which indicate a main axis thereof which must correspond to the steepest axis when said IOL has been implanted.

However, it has been proven that when the incision position is the axis at 45° from the steepest axis or the axis at 45° from the flattest axis, after the incision, both the steepest axis and the flattest axis are angularly displaced from the initial position thereof obtained. Therefore, if the surgeon places the lens on the steepest axis determined before the incision, in reality they would not be placing it on the steepest axis after the incision since, as said, said steepest axis has been displaced by the incision. This implies that, after the intervention, the patient may not have the vision capacity expected from the implantation of the intraocular lens.

For this reason, in a preferred embodiment wherein the incision position is obtained between the axis at 45° from the steepest axis or the axis at 45° from the flattest axis, the orientation of the main axis of the intraocular lens is calculated depending on the angular displacement of the steepest axis which, preferably, is within an interval of [-10, 10] degrees and, more preferably, in an interval of [0.1, 4] degrees.

In this manner, when the surgeon operates with an incision position according to the axis at 45° from the steepest axis or according to the axis at 45° from the flattest axis, they can know the new location of the steepest axis after the incision, which enables the reduction of another source of error, as was the case with such angular displacement of the steepest axis when said incision position was used.

## Claims

1. A computer-implemented method adapted to calculate surgically induced astigmatism when an incision is made in the cornea in order to introduce an intraocular lens with a certain size, **characterised in that** it comprises the steps of:
- establishing an orientation of the steepest axis and an orientation of the flattest axis of the cornea starting from a measurement of the curvature of said cornea by means of an ophthalmic diagnostic device;
- obtaining an incision position with respect to the orientation of a corneal axis;
- obtaining an incision size depending on at least one power of the intraocular lens; wherein obtaining the incision size is carried out depending on the size of a surgical instrument configured to carry out said incision;
- calculating the surgically induced astigmatism depending on the incision position obtained and on the incision size obtained;
- obtaining an incision architecture, which is defined as the number of planes in which the incision can be carried out, wherein the step of calculating the surgically induced astigmatism is further done depending on the incision architecture obtained.

2. The method according to claim 1 wherein, the step of obtaining an incision position with respect to the orientation of a corneal axis, the orientation of the corneal axis is the orientation of the steepest axis or the flattest axis, and wherein the orientation of the corneal axis can further be the orientation of an axis at 45 degrees from the steepest axis or an axis at 45 degrees from the flattest axis, and wherein, if the orientation obtained from the corneal axis is the orientation of an axis at 45 degrees from the steepest axis or an axis at 45 degrees from the flattest axis, performing an estimate of an angular displacement of the steepest axis and/or the flattest axis with respect to an initial position obtained from said flattest axis and steepest axis in an interval of [-10, 10] degrees.

3. The method according to claims 1 and 2 wherein, in the step of calculating the astigmatism, the astigmatism is also calculated depending on an angular sector to which the orientation of the steepest axis or the flattest axis belongs which corresponds to the incision position obtained from among at least the following sectors: [0, 30), [30, 60), [60, 120], (120, 150], (150, 180].

4. The method according to the previous claim, wherein the surgically induced astigmatism calculated for an incision position corresponding to the certain orientation of the steepest axis, a certain incision size and a certain incision architecture, is the same for an incision position corresponding to a symmetrical orientation with respect to a meridian plane which is normal to the orientation of the steepest axis and which passes through the intersection of the flattest axis and the steepest axis.

5. The method according to claim 3, wherein the surgically induced astigmatism calculated for an incision position corresponding to the certain orientation of the flattest axis, a certain incision size and a certain incision architecture, is the same for an incision position corresponding to a symmetrical orientation with respect to a meridian plane which is normal to the orientation of the flattest axis and which passes through the intersection of the flattest axis and the steepest axis.

6. The method according to any of claims 1 to 5 wherein, in the step of obtaining an architecture of the incision, an incision architecture of at least one incision plane is determined.

7. The method according to any of the previous claims wherein, in the step of calculating the surgically induced astigmatism depending on the incision position obtained, the certain incision size is within an interval of 1.8 mm to 3 mm, preferably, the certain incision size is chosen from among any of the following sizes: 2 mm, 2.2 mm and 2.8 mm, wherein the surgically induced astigmatism calculated for the incision size of 2 mm and the surgically induced astigmatism calculated for the incision size of 2.8 mm differ by a maximum of 0.5 D, and wherein, for a certain incision position and a certain incision architecture, the surgically induced astigmatism calculated for the smallest incision size and for the largest incision size differ by a maximum of 0.7 D.

8. The method according to claim 7 when claim 7 depends on claim 6 and said claim 6 depends on any of claims 2 to 5 wherein, if the incision position obtained corresponds to the orientation of the steepest axis and the incision architecture obtained has at least two planes, the surgically induced astigmatism calculated is negative, with a maximum absolute value of 0.75 D; or if the incision position obtained corresponds to the orientation of the flattest axis and the incision architecture obtained has at least two planes, the surgically induced astigmatism calculated is positive, with a maximum value of 0.55D.

9. The method according to claim 7, when claim 7 depends on claim 6 and said claim 6 depends on any of claims 2 to 5 wherein, if the incision position obtained corresponds to the steepest axis and said steepest axis is located within the angular sector of [0, 30) degrees or (150 to 180] degrees and the incision architecture obtained has at least two planes, the surgically induced astigmatism calculated is:
- 0.0 D for an incision size of 2.0 mm and 2.2 mm; and
- -0.15 D for a lens incision size of 2.8 mm.

10. The method according to claim 7, when claim 7 depends on claim 6 and said claim 6 depends on any of claims 2 to 5 wherein, if the incision position obtained corresponds to the flattest axis and said flattest axis is located within the angular sector of [0, 30) degrees or (150 to 180] degrees and the incision architecture obtained has at least two planes, the surgically induced astigmatism calculated is 0.0 D for an incision size of 2.0 mm, 2.2 mm and 2.8 mm.

11. The method according to claim 7, when claim 7 depends on claim 6 and said claim 6 depends on any of claims 2 to 5 wherein, if the incision position obtained corresponds to the steepest axis and said steepest axis is located within the angular sector of [30, 60) degrees or (120 to 150] degrees and the incision architecture obtained has at least two planes, the surgically induced astigmatism calculated is:
- 0.00 D for an incision size of 2.0 mm;
- -0.20 D for an incision size of 2.2 mm; and
- -0.40 D for an incision size of 2.8 mm.

12. The method according to claim 7, when claim 7 depends on claim 6 and said claim 6 depends on any of claims 2 to 5 wherein, if the incision position obtained corresponds to the flattest axis and said flattest axis is located within the angular sector of [30, 60) degrees or (120 to 150] degrees and the incision architecture obtained has at least two planes, the surgically induced astigmatism calculated is:
- 0.00 D for an incision size of 2.0 mm;
- 0.15 D for an incision size of 2.2 mm; and
- 0.30 D for an incision size of 2.8 mm.

13. The method according to claim 7, when claim 7 depends on claim 6 and said claim 6 depends on any of claims 2 to 5 wherein, if the incision position obtained corresponds to the steepest axis and said steepest axis is located within the angular sector of [60, 120] degrees and the incision architecture obtained has at least two planes, the surgically induced astigmatism calculated is:
- -0.25 D for an incision size of 2.0 mm;
- -0.50 D for an incision size of 2.2 mm; and
- -0.75 D for an incision size of 2.8 mm.

14. The method according to claim 7, when claim 7 depends on claim 6 and said claim 6 depends on any of claims 2 to 5 wherein, if the incision position obtained corresponds to the flattest axis and said flattest axis is located within the angular sector of [60, 120] degrees and the incision architecture obtained has at least two planes, the surgically induced astigmatism calculated is:
- 0.20 D for an incision size of 2.0 mm;
- 0.35 D for an incision size of 2.2 mm; and
- 0.55 D for an incision size of 2.8 mm.

15. The method, according to claim 1, wherein, in the step of calculating the astigmatism, if the incision architecture obtained has one plane, the astigmatism calculated has an absolute maximum value of 0.2 D.

## Patentansprüche

1. Computerimplementiertes Verfahren, das dazu angepasst ist, chirurgisch induzierten Astigmatismus zu berechnen, wenn ein Einschnitt in der Hornhaut gemacht wird, um eine Intraokularlinse mit einer gewissen Größe einzuführen, **dadurch gekennzeichnet, dass** es folgende Schritte umfasst:
- Festlegen einer Orientierung der steilsten Achse und einer Orientierung der flachsten Achse der Hornhaut ausgehend von einer Messung der Krümmung der Hornhaut mittels einer ophthalmischen Diagnosevorrichtung;
- Erhalten einer Einschnittposition in Bezug auf die Orientierung einer Hornhautachse;
- Erhalten einer Einschnittgröße in Abhängigkeit von mindestens einer Brechkraft der Intraokularlinse; wobei das Erhalten der Einschnittgröße in Abhängigkeit von der Größe eines chirurgischen Instruments erfolgt, das dazu konfiguriert ist, den Einschnitt durchzuführen;
- Berechnen des chirurgisch induzierten Astigmatismus in Abhängigkeit von der erhaltenen Einschnittposition und der erhaltenen Einschnittgröße;
- Erhalten einer Einschnittarchitektur, die als die Anzahl der Ebenen definiert ist, in denen der Einschnitt durchgeführt werden kann, wobei der Schritt des Berechnens des chirurgisch induzierten Astigmatismus ferner in Abhängigkeit von der erhaltenen Einschnittarchitektur durchgeführt wird.

2. Verfahren nach Anspruch 1, wobei im Schritt des Erhaltens einer Einschnittposition in Bezug auf die Orientierung einer Hornhautachse die Orientierung der Hornhautachse die Orientierung der steilsten Achse oder der flachsten Achse ist, und wobei die Orientierung der Hornhautachse ferner die Orientierung einer Achse bei 45 Grad von der steilsten Achse oder einer Achse bei 45 Grad von der flachsten Achse sein kann, und wobei, falls die von der Hornhautachse erhaltene Orientierung die Orientierung einer Achse bei 45 Grad von der steilsten Achse oder einer Achse bei 45 Grad von der flachsten Achse ist, eine Schätzung einer Winkelverschiebung der steilsten Achse und/oder der flachsten Achse in Bezug auf eine von der flachsten Achse und der steilsten Achse erhaltene Ausgangsposition in einem Intervall von [-10, 10] Grad durchgeführt wird.

3. Verfahren nach Anspruch 1 und 2, wobei im Schritt des Berechnens des Astigmatismus der Astigmatismus auch in Abhängigkeit von einem Winkelsektor berechnet wird, zu dem die Orientierung der steilsten Achse oder der flachsten Achse gehört, die der Einschnittposition entspricht, die aus mindestens den folgenden Sektoren erhalten wird: [0, 30), [30, 60), [60, 120], (120, 150], (150, 180].

4. Verfahren nach dem vorhergehenden Anspruch, wobei der chirurgisch induzierte Astigmatismus, der für eine Einschnittposition berechnet wird, die einer gewissen Orientierung der steilsten Achse, einer gewissen Einschnittgröße und einer gewissen Einschnittarchitektur entspricht, derselbe ist für eine Einschnittposition, die einer symmetrischen Orientierung in Bezug auf eine Meridianebene entspricht, die normal zur Orientierung der steilsten Achse ist und die durch den Schnittpunkt der flachsten Achse und der steilsten Achse verläuft.

5. Verfahren nach Anspruch 3, wobei der chirurgisch induzierte Astigmatismus, der für eine Einschnittposition berechnet wird, die der gewissen Orientierung der flachsten Achse, einer gewissen Einschnittgröße und einer gewissen Einschnittarchitektur entspricht, derselbe ist für eine Einschnittposition, die einer symmetrischen Orientierung in Bezug auf eine Meridianebene entspricht, die normal zur Orientierung der flachsten Achse ist und die durch den Schnittpunkt der flachsten Achse und der steilsten Achse verläuft.

6. Verfahren nach einem der Ansprüche 1 bis 5, wobei im Schritt des Erhaltens einer Architektur des Einschnitts eine Einschnittarchitektur von mindestens einer Einschnittebene bestimmt wird.

7. Verfahren nach einem der vorhergehenden Ansprüche, wobei im Schritt des Berechnens des chirurgisch induzierten Astigmatismus in Abhängigkeit von der erhaltenen Einschnittposition die gewisse Einschnittgröße innerhalb eines Intervalls von 1,8 mm bis 3 mm liegt, wobei die gewisse Einschnittgröße vorzugsweise aus einer der folgenden Größen ausgewählt wird: 2 mm, 2,2 mm und 2,8 mm, wobei sich der für die Einschnittgröße von 2 mm berechnete chirurgisch induzierte Astigmatismus und der für die Einschnittgröße von 2,8 mm berechnete chirurgisch induzierte Astigmatismus um maximal 0,5 D unterscheiden, und wobei sich für eine gewisse Einschnittposition und eine gewisse Einschnittarchitektur der für die kleinste Einschnittgröße berechnete chirurgisch induzierte Astigmatismus und der für die größte Einschnittgröße berechnete Astigmatismus um maximal 0,7 D unterscheiden.

8. Verfahren nach Anspruch 7, wenn Anspruch 7 von Anspruch 6 abhängig ist und Anspruch 6 von einem der Ansprüche 2 bis 5 abhängig ist, wobei, falls die erhaltene Einschnittposition der Orientierung der steilsten Achse entspricht und die erhaltene Einschnittarchitektur mindestens zwei Ebenen aufweist, der berechnete chirurgisch induzierte Astigmatismus negativ ist, mit einem maximalen Absolutwert von 0,75 D; oder falls die erhaltene Einschnittposition der Orientierung der flachsten Achse entspricht und die erhaltene Einschnittarchitektur mindestens zwei Ebenen aufweist, der berechnete chirurgisch induzierte Astigmatismus positiv ist, mit einem Maximalwert von 0,55 D.

9. Verfahren nach Anspruch 7, wenn Anspruch 7 von Anspruch 6 abhängig ist und Anspruch 6 von einem der Ansprüche 2 bis 5 abhängig ist, wobei, falls die erhaltene Einschnittposition der steilsten Achse entspricht und die steilste Achse innerhalb des Winkelsektors von [0, 30) Grad oder (150 bis 180] Grad liegt und die erhaltene Einschnittarchitektur mindestens zwei Ebenen aufweist, der berechnete chirurgisch induzierte Astigmatismus folgender ist:
- 0,0 D für eine Einschnittgröße von 2,0 mm und 2,2 mm; und
- -0,15 D für eine Linseneinschnittgröße von 2,8 mm.

10. Verfahren nach Anspruch 7, wenn Anspruch 7 von Anspruch 6 abhängig ist und Anspruch 6 von einem der Ansprüche 2 bis 5 abhängig ist, wobei, falls die erhaltene Einschnittposition der flachsten Achse entspricht und die flachste Achse innerhalb des Winkelsektors von [0, 30) Grad oder (150 bis 180] Grad liegt und die erhaltene Einschnittarchitektur mindestens zwei Ebenen aufweist, der berechnete chirurgisch induzierte Astigmatismus 0,0 D für eine Einschnittgröße von 2,0 mm, 2,2 mm und 2,8 mm beträgt.

11. Verfahren nach Anspruch 7, wenn Anspruch 7 von Anspruch 6 abhängig ist und Anspruch 6 von einem der Ansprüche 2 bis 5 abhängig ist, wobei, falls die erhaltene Einschnittposition der steilsten Achse entspricht und die steilste Achse innerhalb des Winkelsektors von [30, 60) Grad oder (120 bis 150] Grad liegt und die erhaltene Einschnittarchitektur mindestens zwei Ebenen aufweist, der berechnete chirurgisch induzierte Astigmatismus folgender ist:
- 0,00 D für eine Einschnittgröße von 2,0 mm;
- -0,20 D für eine Einschnittgröße von 2,2 mm; und
- -0,40 D für eine Einschnittgröße von 2,8 mm.

12. Verfahren nach Anspruch 7, wenn Anspruch 7 von Anspruch 6 abhängig ist und Anspruch 6 von einem der Ansprüche 2 bis 5 abhängig ist, wobei, falls die erhaltene Einschnittposition der flachsten Achse entspricht und die flachste Achse innerhalb des Winkelsektors von [30, 60) Grad oder (120 bis 150] Grad liegt und die erhaltene Einschnittarchitektur mindestens zwei Ebenen aufweist, der berechnete chirurgisch induzierte Astigmatismus folgender ist:
- 0,00 D für eine Einschnittgröße von 2,0 mm;
- 0,15 D für eine Einschnittgröße von 2,2 mm; und
- 0,30 D für eine Einschnittgröße von 2,8 mm.

13. Verfahren nach Anspruch 7, wenn Anspruch 7 von Anspruch 6 abhängig ist und Anspruch 6 von einem der Ansprüche 2 bis 5 abhängig ist, wobei, falls die erhaltene Einschnittposition der steilsten Achse entspricht und die steilste Achse innerhalb des Winkelsektors von [60, 120] Grad liegt und die erhaltene Einschnittarchitektur mindestens zwei Ebenen aufweist, der berechnete chirurgisch induzierte Astigmatismus folgender ist:
- -0,25 D für eine Einschnittgröße von 2,0 mm;
- -0,50 D für eine Einschnittgröße von 2,2 mm; und
- -0,75 D für eine Einschnittgröße von 2,8 mm.

14. Verfahren nach Anspruch 7, wenn Anspruch 7 von Anspruch 6 abhängig ist und Anspruch 6 von einem der Ansprüche 2 bis 5 abhängig ist, wobei, falls die erhaltene Einschnittposition der flachsten Achse entspricht und die flachste Achse innerhalb des Winkelsektors von [60, 120] Grad liegt und die erhaltene Einschnittarchitektur mindestens zwei Ebenen aufweist, der berechnete chirurgisch induzierte Astigmatismus folgender ist:
- 0,20 D für eine Einschnittgröße von 2,0 mm;
- 0,35 D für eine Einschnittgröße von 2,2 mm; und
- 0,55 D für eine Einschnittgröße von 2,8 mm.

15. Verfahren nach Anspruch 1, wobei im Schritt des Berechnens des Astigmatismus, falls die erhaltene Einschnittarchitektur eine Ebene aufweist, der berechnete Astigmatismus einen absoluten Maximalwert von 0,2 D aufweist.

## Revendications

1. Procédé mis en œuvre par ordinateur adapté pour calculer un astigmatisme induit par la chirurgie lorsqu'une incision est pratiquée dans la cornée afin d'introduire une lentille intraoculaire avec une certaine taille, **caractérisé en ce qu'**il comprend les étapes consistant à :
- établir une orientation de l'axe le plus raide et une orientation de l'axe le plus plat de la cornée à partir d'une mesure de la courbure de ladite cornée au moyen d'un dispositif de diagnostic ophtalmique ;
- obtenir une position d'incision par rapport à l'orientation d'un axe cornéen ;
- obtenir une taille d'incision en fonction d'au moins une puissance de la lentille intraoculaire ; dans lequel l'obtention de la taille de l'incision est effectuée en fonction de la taille d'un instrument chirurgical conçu pour effectuer ladite incision ;
- calculer l'astigmatisme induit par la chirurgie en fonction de la position d'incision obtenue et de la taille d'incision obtenue ;
- obtenir une architecture d'incision, qui est définie comme le nombre de plans dans lesquels l'incision peut être effectuée, dans lequel l'étape de calcul de l'astigmatisme induit par la chirurgie est réalisée en outre en fonction de l'architecture d'incision obtenue.

2. Procédé selon la revendication 1 dans lequel, l'étape d'obtention d'une position d'incision par rapport à l'orientation d'un axe cornéen, l'orientation de l'axe cornéen est l'orientation de l'axe le plus raide ou de l'axe le plus plat, et dans lequel l'orientation de l'axe cornéen peut en outre être l'orientation d'un axe à 45 degrés de l'axe le plus raide ou d'un axe à 45 degrés de l'axe le plus plat, et dans lequel, si l'orientation obtenue à partir de l'axe cornéen est l'orientation d'un axe à 45 degrés de l'axe le plus raide ou d'un axe à 45 degrés de l'axe le plus plat, la réalisation d'une estimation d'un déplacement angulaire de l'axe le plus raide et/ou de l'axe le plus plat par rapport à une position initiale obtenue à partir dudit axe le plus plat et dudit axe le plus raide dans un intervalle de [-10, 10] degrés.

3. Procédé selon les revendications 1 et 2 dans lequel, dans l'étape de calcul de l'astigmatisme, l'astigmatisme est également calculé en fonction d'un secteur angulaire auquel appartient l'orientation de l'axe le plus raide ou de l'axe le plus plat qui correspond à la position d'incision obtenue parmi au moins les secteurs suivants : [0, 30), [30, 60), [60, 120], (120, 150], (150, 180].

4. Procédé selon la revendication précédente, dans lequel l'astigmatisme induit par la chirurgie calculé pour une position d'incision correspondant à la certaine orientation de l'axe le plus raide, une certaine taille d'incision et une certaine architecture d'incision, est le même pour une position d'incision correspondant à une orientation symétrique par rapport à un plan méridien qui est normal à l'orientation de l'axe le plus raide et qui passe par l'intersection de l'axe le plus plat et de l'axe le plus raide.

5. Procédé selon la revendication 3, dans lequel l'astigmatisme induit par la chirurgie calculé pour une position d'incision correspondant à la certaine orientation de l'axe le plus plat, une certaine taille d'incision et une certaine architecture d'incision, est le même pour une position d'incision correspondant à une orientation symétrique par rapport à un plan méridien qui est normal à l'orientation de l'axe le plus plat et qui passe par l'intersection de l'axe le plus plat et de l'axe le plus raide.

6. Procédé selon l'une quelconque des revendications 1 à 5 dans lequel, dans l'étape d'obtention d'une architecture de l'incision, une architecture d'incision d'au moins un plan d'incision est déterminée.

7. Procédé selon l'une quelconque des revendications précédentes dans lequel, dans l'étape de calcul de l'astigmatisme induit par la chirurgie en fonction de la position d'incision obtenue, la certaine taille d'incision est au sein d'un intervalle de 1,8 mm à 3 mm, de préférence, la certaine taille d'incision est choisie parmi l'une quelconque des tailles suivantes : 2 mm, 2,2 mm et 2,8 mm, dans lequel l'astigmatisme induit par la chirurgie calculé pour la taille d'incision de 2 mm et l'astigmatisme induit par la chirurgie calculé pour la taille d'incision de 2,8 mm diffèrent d'un maximum de 0,5 D, et dans lequel, pour une certaine position d'incision et une certaine architecture d'incision, l'astigmatisme induit par la chirurgie calculé pour la plus petite taille d'incision et pour la plus grande taille d'incision diffère d'un maximum de 0,7 D.

8. Procédé selon la revendication 7, lorsque la revendication 7 dépend de la revendication 6 et que ladite revendication 6 dépend de l'une quelconque des revendications 2 à 5, dans lequel, si la position d'incision obtenue correspond à l'orientation de l'axe le plus raide et l'architecture d'incision obtenue a au moins deux plans, l'astigmatisme induit par la chirurgie calculé est négatif, avec une valeur absolue maximale de 0,75 D ; ou si la position d'incision obtenue correspond à l'orientation de l'axe le plus plat et l'architecture d'incision obtenue a au moins deux plans, l'astigmatisme induit par la chirurgie calculé est positif, avec une valeur maximale de 0,55 D.

9. Procédé selon la revendication 7, lorsque la revendication 7 dépend de la revendication 6 et que ladite revendication 6 dépend de l'une quelconque des revendications 2 à 5, dans lequel, si la position d'incision obtenue correspond à l'axe le plus raide et ledit axe le plus raide est situé au sein du secteur angulaire de [0, 30) degrés ou de (150 à 180] degrés et l'architecture d'incision obtenue a au moins deux plans, l'astigmatisme induit par la chirurgie calculé est :
- 0,0 D pour une taille d'incision de 2,0 mm et 2,2 mm ; et
- -0,15 D pour une taille d'incision de lentille de 2,8 mm.

10. Procédé selon la revendication 7, lorsque la revendication 7 dépend de la revendication 6 et que ladite revendication 6 dépend de l'une quelconque des revendications 2 à 5, dans lequel, si la position d'incision obtenue correspond à l'axe le plus plat et ledit axe le plus plat est situé au sein du secteur angulaire de [0, 30) degrés ou de (150 à 180] degrés et l'architecture d'incision obtenue a au moins deux plans, l'astigmatisme induit par la chirurgie calculé est de 0,0 D pour une taille d'incision de 2,0 mm, 2,2 mm et 2,8 mm.

11. Procédé selon la revendication 7, lorsque la revendication 7 dépend de la revendication 6 et que ladite revendication 6 dépend de l'une quelconque des revendications 2 à 5, dans lequel, si la position d'incision obtenue correspond à l'axe le plus raide et ledit axe le plus raide est situé au sein du secteur angulaire de [30, 60) degrés ou de (120 à 150] degrés et l'architecture d'incision obtenue a au moins deux plans, l'astigmatisme induit par la chirurgie calculé est :
- 0,00 D pour une taille d'incision de 2,0 mm ;
- -0,20 D pour une taille d'incision de 2,2 mm ; et
- -0,40 D pour une taille d'incision de 2,8 mm.

12. Procédé selon la revendication 7, lorsque la revendication 7 dépend de la revendication 6 et que ladite revendication 6 dépend de l'une quelconque des revendications 2 à 5, dans lequel, si la position d'incision obtenue correspond à l'axe le plus plat et ledit axe le plus plat est situé au sein du secteur angulaire de [30, 60) degrés ou de (120 à 150] degrés et l'architecture d'incision obtenue a au moins deux plans, l'astigmatisme induit par la chirurgie calculé est :
- 0,00 D pour une taille d'incision de 2,0 mm ;
- 0,15 D pour une taille d'incision de 2,2 mm ; et
- 0,30 D pour une taille d'incision de 2,8 mm.

13. Procédé selon la revendication 7, lorsque la revendication 7 dépend de la revendication 6 et que ladite revendication 6 dépend de l'une quelconque des revendications 2 à 5, dans lequel, si la position d'incision obtenue correspond à l'axe le plus raide et ledit axe le plus raide est situé au sein du secteur angulaire de [60, 120] degrés et l'architecture d'incision obtenue a au moins deux plans, l'astigmatisme induit par la chirurgie calculé est :
- -0,25 D pour une taille d'incision de 2,0 mm ;
- -0,50 D pour une taille d'incision de 2,2 mm ; et
- -0,75 D pour une taille d'incision de 2,8 mm.

14. Procédé selon la revendication 7, lorsque la revendication 7 dépend de la revendication 6 et que ladite revendication 6 dépend de l'une quelconque des revendications 2 à 5, dans lequel, si la position d'incision obtenue correspond à l'axe le plus plat et ledit axe le plus plat est situé au sein du secteur angulaire de [60, 120] degrés et l'architecture d'incision obtenue a au moins deux plans, l'astigmatisme induit par la chirurgie calculé est :
- 0,20 D pour une taille d'incision de 2,0 mm ;
- 0,35 D pour une taille d'incision de 2,2 mm ; et
- 0,55 D pour une taille d'incision de 2,8 mm.

15. Procédé, selon la revendication 1, dans lequel, dans l'étape de calcul de l'astigmatisme, si l'architecture d'incision obtenue a un plan, l'astigmatisme calculé a une valeur maximale absolue de 0,2 D.
